# EUROPÄISCHE PATENTSCHRIFT

(11) **EP 2 470 580 B1**
(45) Veröffentlichungstag und Bekanntmachung des Hinweises auf die Patenterteilung: **26.02.2014**
(21) Anmeldenummer: 10745269.0
(22) Anmeldetag: 26.08.2010
(51) Int. Cl.: C08G 18/73, A61L 15/26, C08G 18/10, C08G 18/12

(54) **HYDROPHILE, ALIPHATISCHE POLYURETHAN-SCHÄUME**
HYDROPHILIC ALIPHATIC POLYURETHANE FOAMS
MOUSSES DE POLYURÉTHANE ALIPHATIQUES HYDROPHILES

(30) Priorität: 29.08.2009 EP 09011102
(43) Veröffentlichungstag der Anmeldung: 04.07.2012
(73) Patentinhaber: Bayer Intellectual Property GmbH, 40789 Monheim (DE)
(72) Erfinder: DÖRR, Sebastian, 40593 Düsseldorf (DE); NIESTEN, Meike, 51061 Köln (DE)
(74) Vertreter: BIP Patents
(86) Internationale Anmeldenummer: PCT/EP2010/062477
(87) Internationale Veröffentlichungsnummer: WO 2011/023762

(56) Entgegenhaltungen:
- EP-A1- 0 299 122
- WO-A1-93/04101
- US-A1- 2009 118 387

## Beschreibung

Die Erfindung betrifft hydrophile, aliphatische Polyurethan-Schäume, die durch Reaktion von speziellen monomerenarmen Präpolymeren und hydrophilen Polyisocyanaten in Gegenwart von Wasser zugänglich sind. Aufgrund ihrer Absorptionseigenschaften sind die Polyurethan-Schäume insbesondere zur Herstellung von Wundauflagen, kosmetischen Artikeln oder Inkontinenzprodukten geeignet.

Die EP-A 949285 beschreibt die Reaktion von Polyisocyanaten mit primären Diaminen, niedermole-kularen Polyolen und hochmolekularen Polyolen. Bei dieser Reaktion ist nicht auszuschließen, dass erhebliche Teile der isocyanatreaktiven Substanzen nicht umgesetzt werden und anschließend aus dem hydrophilen Schaum extrahierbar sind.

Die GB 1571730 beschreibt die Reaktion von Diisocyanaten mit hohem Dampfdruck wie Isophoron-diisocyanat (IPDI) und Bis(isocyanatocyclohexyl)methan (HMDI) mit Polyolen. Auch hier bleiben nicht umgesetzte Komponenten zurück. Ferner ist das Arbeiten mit freien, nicht derivatisierten Diisocyanaten aus Sicht der Arbeitshygiene problematisch. In der WO 2004013215 werden ebenfalls leicht flüchtige Diisocyanate umgesetzt.

Die W02003/097727, US 5065752 und US 5064653 beschreiben Schaumbildungsreaktion von Präpolymeren in Gegenwart von Acrylamid-Acrylsäure-Copolymeren. Diese Produkte sind chemisch nicht angekoppelt und können komplett extrahiert werden, was nicht wünschenswert ist.

In der US 3903232 und US 388941 werden Präpolymere mit Polyethern umgesetzt. Auch hier besteht die Gefahr des Auftretens nicht angekoppelter Polyole. Die US 5296518 beschreibt ebenso die Umsetzung von Präpolymeren mit Polyethern, wobei drei unterschiedliche Polyole eingesetzt werden, was die Wirtschaftlichkeit dieses Verfahrens in Frage stellt. Zudem lässt sich mit dem dort beschriebenen Verfahren nicht sicherstellen, dass keine niedermolekularen Isocyanate im Gemisch verbleiben, was nicht wünschenswert ist. Die Herstellung der Präpolymere erfordert zumeist unwirtschaftlich lange Reaktionszeiten.

Die europäische Patentanmeldung mit der Anmeldenummer 08012372.2 beschreibt hydrophile aliphatische Polyurethanschäume basierend auf monomerarmen Präpolymeren. Die Hydrophile der resultierenden Schäume ist jedoch begrenzt. Aus der EP 08012372.2 sind keine Polyurethanschäume bekannt, die zusätzliche hydrophile Polyisocyanate enthalten.

WO 93/04101 offenbart hydrophile Polyurethan-Schäume, die als Wundauflagen verwendet werden können Auf Seiten 17-19 wird ein Blend von zwei Isocyanat-Präpolymeren beschneben, wobei zur Herstellung des zweiten Isocyanat-Präpolymers ein aromatisches (und nicht aliphatisch) Isocyanat verwendet Außerdem wird der Monomergehalt an Isocyanate nicht offenbart

Bei den bekannten Polyurethanschäumen ist entweder das Aufnahmevermögen für hydrophile Flüssigkeiten nicht ausreichend oder sie enthalten extrahierbare Substanzen, die mit Hinblick auf ihre Zellverträglichkeit als problematisch einzustufen sind.

Aufgabe der vorliegenden Erfindung war daher ein Verfahrens zur Herstellung von PolyurethanSchäumen bereit zu stellen, die nur wenig extrahierbare Bestandteile aufweisen, zellverträglich sind, eine große Menge hydrophiler Flüssigkeiten wie physiologische Salzlösung oder Wundflüssigkeit schnell aufnehmen können und derart verformbar sind, dass sie sich beispielsweise der Form einer Wundoptimal anpassen können.

Diese Aufgabe wird erfindungsgemäß durch ein Verfahren zur Herstellung hydrophiler, aliphatischer Polyurethan-Schäume gelöst, bei dem Zusammensetzungen umfassend
A) isocyanatfunktionelle Präpolymere mit einem Gewichtsanteil an niedermolekularen, aliphatischen Diisocyanaten mit einer Molmasse von 140 bis 278 g/mol von unter 1,0 Gew.-% bezogen auf das Präpolymer, erhältlich durch Umsetzung von
   A1) niedermolekularen, aliphatischen Diisocyanaten einer Molmasse von 140 bis 278 g/mol mit
   A2) di- bis hexafunktionellen, bevorzugt tri- bis hexafunktionellen Polyalkylenoxiden einer OH-Zahl von 22,5 bis 112 und einem Ethylenoxidanteil von 50 bis 100 mol% bezogen auf die Gesamtmenge der enthaltenen Oxyalkylengruppen,
B) gegebenenfalls heterocyclische, 4-Ring- oder 6-Ring-Oligomere von niedermolekularen, aliphatischen Diisocyanaten mit einer Molmasse von 140 bis 278 g/mol,
C) Wasser,
D) gegebenenfalls Katalysatoren,
E) gegebenenfalls C₈- bis C₂₂-Monocarbonsäuren oder deren Ammonium- oder Alkalisalze oder C₁₂- bis C₄₄-Dicarbonsäuren oder deren Ammonium- oder Alkalisalze,
F) gegebenenfalls Tenside und
G) gegebenenfalls ein- oder mehrwertige Alkohole
H) hydrophile Polyisocyanate erhältlich durch Umsetzung von
   H1) niedermolekularen, aliphatischen Diisocyanaten einer Molmasse von 140 bis 278 g/mol und / oder daraus herstellbaren Polyisocyanaten mit einer Isocyanatfunktionalität von 2 bis 6 mit
   H2) monofunktionellen Polyalkylenoxiden einer OH-Zahl von 10 bis 250 und einem Ethylenoxidanteil von 50 bis 100 mol% bezogen auf die Gesamtmenge der enthaltenen Oxyalkylengruppen.
bereitgestellt, aufgeschäumt und ausgehärtet werden.

Bevorzugt weisen die eingesetzten Präpolymere A) einen Restmonomergehalt von unter 0,5 Gew.-% bezogen auf das Präpolymer auf. Dieser Gehalt kann durch entsprechend gewählte Einsatzmengen der Diisocyanate A1) und der Polyalkylenoxide A2) erreicht werden. Bevorzugt ist jedoch der Einsatz des Diisocyanats A1) im Überschuss und mit anschließender, bevorzugt destillativer, Abtren-nung nicht umgesetzter Monomere.

Bei der Herstellung der isocyanatfunktionellen Präpolymere A) wird das Verhältnis der Polyalkylenoxide A2) zu den niedermolekularen, aliphatischen Diisocyanaten A1) typischerweise so eingestellt, dass auf 1 Mol OH-Gruppen des Polyalkylenoxide A2) 2 bis 20 Mol, bevorzugt mit 2 bis 10 Mol und besonders bevorzugt 5 bis 10 Mol NCO-Gruppen des niedermolekularen, aliphatischen Diisocyanats A1) kommen.

Die Umsetzung kann in Gegenwart von Urethanisierungskatalysatoren wie Zinnverbindungen, Zinkverbindungen, Aminen, Guanidinen oder Amidinen, oder in Gegenwart von Allophanatisierungskata-lysatoren wie Zinkverbindungen erfolgen.

Die Umsetzung erfolgt typischerweise bei 25 bis 140 °C, bevorzugt 60 bis 100 °C.

Wurde mit überschüssigem Isocyanat gearbeitet, so erfolgt anschließend die Entfernung des Überschusses an niedermolekularem, aliphatischen Diisocyanat bevorzugt durch Dünnschichtdestillation.

Vor, während und nach der Reaktion oder destillativen Abtrennung des Diisocyanatüberschusses können saure oder alkylierende Stabilisatoren, wie Benzoylchlorid, Isophthaloylchlorid, Methyltosylat, Chlorpropionsäure, HCl oder Antioxidantien wie Di-*tert*-butylkresol oder Tocopherol zugesetzt werden.

Der NCO-Gehalt der isocyanatfunktionellen Präpolymere A) beträgt bevorzugt 1,5 bis 4,5 Gew.-%, besonders bevorzugt 1,5 bis 3,5 Gew.-% und ganz besonders bevorzugt 1,5 bis 3,0 Gew.-%. Beispiele für niedermolekulare, aliphatische Diisocyanate der Komponente A1) sind Hexamethylen-diisocyanat (HDI), Isophorondiisocyanat (IPDI), Butylendiisocyanat (BDI), Bisisocyanatocyclohe-xylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat (HDI), Isophorondiisocyanat (IPDI), Butylendiisocyanat (BDI) und Bis(isocyanatocyclohexyl)methan (HMDI) bevorzugt sind. Besonders bevorzugt sind BDI, HDI, IPDI, ganz besonders bevorzugt Hexamethylendiisocyanat und Isophorondiisocyanat.

Polyalkylenoxide der Komponente A2) sind bevorzugt Copolymere aus Ethylenoxid und Propylenoxid mit einem Ethylenoxidgehalt, bezogen auf die Gesamtmenge der enthaltenen Oxyalkylengruppen, von 50 bis 100 mol%, bevorzugt von 60 bis 85 mol%, gestartet auf Polyolen oder Aminen. Geeignete Starter dieser Art sind Glycerin, Trimethylolpropan (TMP), Sorbit, Pentaerythrit, Triethanolamin, Ammoniak oder Ethylendiamin.

Die Polyalkylenoxide der Komponente A2) besitzen typischerweise zahlenmittlere Molekulargewichte von 1000 bis 15000 g/mol, bevorzugt von 3000 bis 8500 g/mol.

Ferner besitzen die Polyalkylenoxide der Komponente A2) OH-Funktionalitäten von 2 bis 6, bevorzugt von 3 bis 6, besonders bevorzugt von 3 bis 4.

Gegebenenfalls einzusetzende Verbindungen der Komponente B) sind heterocyclische, 4-Ring- oder 6-Ring-Oligomere von niedermolekularen, aliphatischen Diisocyanaten mit einer Molmasse von 140 bis 278 g/mol wie Isocyanurate, Iminooxadiazindione oder Uretdione der vorgenannten niedermolekularen, aliphatischen Diisocyanate. Bevorzugt sind heterocyclische 4-Ring-Oligomere wie Uretdione.

Der durch den Einsatz der Komponente B) erhöhte Gehalt an Isocyanatgruppen sorgt für ein besseres Aufschäumen, da bei der Isocyanat-Wasser-Reaktion mehr CO₂ gebildet wird.

Das als Komponente C) einzusetzende Wasser kann als solches, als Kristallwasser eines Salzes, als Lösung in einem dipolar-aprotischen Lösungsmittel oder auch als Emulsion eingesetzt werden. Bevorzugt wird das Wasser als solches oder in einem dipolar-aprotischen Lösungsmittel eingesetzt. Ganz besonders bevorzugt wird das Wasser als solches eingesetzt.

Zur Beschleunigung der Urethanbildung können in Komponente D) Katalysatoren eingesetzt werden. Dabei handelt es sich typischerweise um die dem Fachmann aus der Polyurethantechnologie bekannten Verbindungen. Bevorzugt sind hier Verbindungen der Gruppe bestehend aus katalytisch aktiven Metallsalzen, Aminen, Amidinen und Guanidinen. Beispielhaft zu nennen sind Zinndibutyldilaurat (DBTL), Zinnacetat, 1,8-Diazabi-cyclo[5.4.0]undecen-7 (DBU), 1,5-Diazabicyclo[4.3.0]nonen-5 (DBN), 1,4-Diazabicy-clo[3.3.0]octen-4 (DBO), N-Ethylmorpholin (NEM), Triethylendiamin (DABCO), Pentamethylguanidin (PMG), Tetrametylguanidin (TMG), Cyclotetramethylguanidin (TMGC), n-Decyl-tetramethylguanidin (TMGD), n-Dodecyltetramethylguanidin (TMGDO), Dimethylaminoethyltetramethylguanidin (TMGN), 1,1,4,4,5,5-Hexamethylisobiguanidin (HMIB), Phenyltetramethylguanidin (TMGP) und Hexamethylenoctamethylbiguanidin (HOBG).

Bevorzugt ist der Einsatz von Aminen, Amidinen, Guanidinen oder deren Mischungen als Katalysatoren der Komponente D) Bevorzugt ist auch die Verwendung von 1,8-Diazabi-cyclo[5.4.0]undecen7 (DBU).

In einer besonders bevorzugten Ausführungsform der Erfindung wird ganz auf Katalysatoren verzichtet.

Gegebenenfalls können als Komponente E) Ammonium- und Alkalisalze von C₈- bis C₂₂-Monocarboxylaten oder deren freien Carbonsäuren oder C₁₂- bis C₄₄-Dicarboxylaten oder deren freien Dicarbonsäuren, bevorzugt Kalium- oder Natriumsalze von C₈- bis C₂₂-Monocarboxylaten oder C₁₂- bis C₄₄-Dicarboxylaten und besonders bevorzugt Natriumsalze von C₈- bis C₂₂-Monocarboxylaten eingesetzt werden.

Beispiele geeigneter Verbindungen der Komponente E) sind die Ammonium-, Na-, Li- oder K-Salze von Ethylhexansäure, Octansäure, Decansäure, Dodecansäure, Palmitinsäure, Stearinsäure, den Octadecensäuren, den Octadecadiensäuren, den Octadecatriensäuren, Isostearinsäure, Erucasäure, Abietinsäure und ihren Hydrierungsprodukten. Beispiele für C₁₂- bis C₄₄-Dicarbonsäuren bzw. die daraus abgeleiteten Ammonium- und Alkalisalze sind Dodecandisäure, Dodecenyl-, Tetradecenyl-, Hexadecenyl- und Octadecenyl-Bernsteinsäure, C₃₆- und C₄₄-Dimerfettsäuren und ihre Hydrierungsprodukte sowie die entsprechenden Ammonium-, Na-, Li- oder K-Salze dieser Dicarbonsäuren.

Zur Verbesserung der Schaumbildung, Schaumstabilität oder der Eigenschaften des resultierenden Polyurethan-Schaums können Verbindungen der Komponente F) eingesetzt werden, wobei solche Additive grundsätzlich alle an sich bekannten anionischen, kationischen, amphoteren und nichtionischen Tenside sowie Mischungen hieraus sein können. Bevorzugt werden Alkylpolyglycoside, EO/PO-Blockcopolymere, Alkyl- oder Arylalkoxylate, Siloxanalkoxylate, Ester der Sulfobemsteinsäure und/oder Alkali- oder Erdalkalimetallalkanoate eingesetzt. Besonders bevorzugt werden EO/PO-Blockcopolymere eingesetzt. Bevorzugt werden allein die EO/PO-Blockcopolymere als Komponente F) eingesetzt.

Zudem können zur Verbesserung der Schaumeigenschaften des resultierenden Polyurethan-Schaums Verbindungen der Komponente G) verwendet werden. Hierbei handelt es sich um grundsätzlich alle dem Fachmann an sich bekannten ein- und mehrwertigen Alkohole sowie Mischungen hieraus. Dies sind ein- oder mehrwertige Alkohole oder Polyole, wie Ethanol, Propanol, Butanol, Decanol, Tridecanol, Hexadecanol, Ethylenglykol, Neopentylglykol, Butandiol, Hexandiol, Decandiol, Trimethylolpropan, Glycerin, Pentaerythrit, monofunktionelle Polyetheralkohole und Polyesteralkohole, Polyetherdiole und Polyesterdiole.

Bei der Herstellung der hydrophilen Polyisocyanate H) wird das Verhältnis der monofunktionellen Polyalkylenoxide H2) zu den niedermolekularen, aliphatischen Diisocyanaten H1) typischerweise so eingestellt, dass auf 1 Mol OH-Gruppen der monofunktionellen Polyalkylenoxide 1,25 bis 15 Mol, bevorzugt mit 2 bis 10 Mol und besonders bevorzugt 2 bis 6 Mol NCO-Gruppen des niedermolekularen, aliphatischen Diisocyanats H1) kommen. Anschließend erfolgt die Allophanatisierung bzw. Biurethisierung und/oder Isocyanuratbildung bzw. Uretdionbildung. Werden die Polyalkylenoxide H2) über Urethangruppen an die aliphatischen Diisocyanate H1) gebunden, findet bevorzugt im Anschluss eine Allophanatisierung statt. Weiterhin ist bevorzugt, dass Isocyanurat-Struktureinheiten gebildet werden.

Eine alternative Herstellung der hydrophilen Polyisocyanate H) erfolgt typischerweise durch Umsetzung von 1 Mol OH-Gruppen der der monofunktioneller Polyalkylenoxidkomponente H2) mit 1,25 bis 15 Mol, bevorzugt mit 2 bis 10 Mol und besonders bevorzugt 2 bis 6 Mol NCO-Gruppen eines Polyisocyanates H1) mit einer Isocyanatfunktionalität von 2 bis 6, basierend auf aliphatischen Disocyanaten. Beispielhaft für derartige Polyisocyanate H1) sind Biuret-Strukturen, Isocyanurate bzw. Uretdione basierend auf aliphatischen Diisocyanaten. Dabei werden das Polyisocyanat H1) und das Polyalkylenoxid H2) bevorzugt über eine Urethangruppe bzw. eine Harnstoffgruppe miteinander verknüpft, wobei besonders die Verknüpfung über Urethangruppen bevorzugt ist.

Die Umsetzung kann in Gegenwart von Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen erfolgen.

Die Umsetzung erfolgt typischerweise bei 25 bis 140 °C, bevorzugt 60 bis 100 °C.

Wurde mit überschüssigem niedermolekularen Diisocyanat gearbeitet, erfolgt anschließend die Entfernung des Überschusses an niedermolekularem, aliphatischen Diisocyanat bevorzugt durch Dünnschichtdestillation.

Vor, während und nach der Reaktion oder der destillativen Abtrennung des Diisocyanatüberschusses können saure oder alkylierende Stabilisatoren, wie Benzoylchlorid, Isophthaloylchlorid, Methyltosylat, Chlorpropionsäure, HCl oder Antioxidantien, wie Di-*tert*-butylkresol oder Tocopherol zugesetzt werden.

Der NCO-Gehalt der hydrophilen Polyisocyanate H) beträgt bevorzugt 0,3 bis 20 Gew.-%, besonders bevorzugt 2 bis 10 Gew.-% und ganz besonders bevorzugt 3 bis 6 Gew.-%.

Beispiele für niedermolekulare, aliphatische Diisocyanate der Komponente H1) sind Hexamethylendiisocyanat (HDI), Isophorondiisocyanat (IPDI), Butylendiisocyanat (BDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat (HDI), Isophorondiisocyanat (IPDI), Butylendiisocyanat (BDI) und Bis(isocyanatocyclohexyl)methan (HMDI) bevorzugt sind. Besonders bevorzugt sind BDI, HDI, IPDI, ganz besonders bevorzugt Hexamethylendiisocyanat und Isophorondiisocyanat.

Beispiele für höhermolekulare Polyisocyanate H2) sind Polyisocyanate mit einer Isocyanatfunktionalität von 2 bis 6 mit Isocyanurat-, Urethan-, Allophanat-, Biuret-, Iminooxadiazintrion-, Oxadiazintrion- und/oder Uretdiongruppen auf Basis der im vorstehenden Abschnitt genannten aliphatischen und/oder cycloaliphatischen Diisocyanate.

Bevorzugt werden als Komponente H2) höhermolekulare Verbindungen mit Biuret-, Iminooxadiazindion,- Isocyanurat- und/oder Uretdiongruppen auf Basis von Hexamethylendiisocyanat, Isophorondiisocyanat und/oder 4,4'-Diisocyanatodicyclohexylmethan eingesetzt. Weiter bevorzugt sind Isocyanurate. Ganz besonders bevorzugt sind Strukturen auf Basis von Hexamethylendiisocyanat.

Die monofunktionellen Polyalkylenoxiden H2) weisen eine OH-Zahl von 15 bis 250, bevorzugt von 28 bis 112, und einem Ethylenoxidanteil von 50 bis 100 mol%, bevorzugt von 60 bis 100 mol%, bezogen auf die Gesamtmenge der enthaltenen Oxyalkylengruppen auf.

Unter monofunktionellen Polyalkylenoxiden im Sinne der Erfindung sind Verbindungen zu verstehen, die nur eine isocyanatreaktive Gruppe, d.h. eine Gruppe, die mit einer NCO-Gruppe reagieren kann, aufweisen.

Die Herstellung von Polyalkylenoxiden H2) durch Alkoxylierung geeigneter Startermoleküle ist literaturbekannt (z.B. Ullmanns Encyclopädie der technischen Chemie, 4. Auflage, Band 19, Verlag Chemie, Weinheim S. 31-38). Geeignete Startermoleküle sind insbesondere gesättigte Monoalkohole wie Methanol, Ethanol, n-Propanol, Isopropanol, n-Butanol, Isobutanol, sec-Butanol, Diethylenglykolmonobutylether sowie aromatische Alkohole wie Phenol oder Monoamine wie Diethylamin. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Die monofunktionellen Polyalkylenoxide H2) besitzen typischerweise zahlenmittlere Molekulargewichte von 220 bis 3700 g/mol, bevorzugt von 500 bis 2800 g/mol.

Die monofunktionellen Polyalkylenoxide H2) besitzen bevorzugt eine OH-Gruppe als isocyanatreaktive Gruppe.

Typischerweise werden die Komponenten A) bis H) in folgenden Mengen eingesetzt:
100 Gewichtsteile isocyanatfunktioneller Präpolymere A)
0 bis 30 Gewichtsteile heterocyclischer Oligomere B)
0,1 bis 200 Gewichtsteile Wasser C)
0 bis 1 Gewichtteile an Katalysatoren D)
0 bis 5 Gewichtteile an C₈- bis C₁₂-Monocarbonsäuren oder deren Ammonium- oder Alkalisalze oder C₁₂- bis C₄₄-Dicarbonsäuren oder deren Ammonium- oder Alkalisalze E)
0 bis 10 Gewichtsteile Tenside F)
0 bis 20 Gewichtsteile Alkohole G)
5 bis 250 Gewichtsteilen hydrophile Polyisocyanatkomponente H)

Bevorzugt werden die Komponenten A) bis H) in folgenden Mengen eingesetzt:
100 Gewichtsteile isocyanatfunktioneller Präpolymere A)
1 bis 30 Gewichtsteile heterocyclischer Oligomere B)
0,1 bis 100 Gewichtsteile Wasser C)
0 bis 1 Gewichtteile an Katalysatoren D)
0,01 bis 5 Gewichtteile an C₈- bis C₁₂-Monocarbonsäuren oder deren Ammonium- oder Alkalisalze oder C₁₂- bis C₄₄-Dicarbonsäuren oder deren Ammonium- oder Alkalisalze E)
0 bis 5 Gewichtsteile Tenside F)
0 bis 10 Gewichtsteile Alkohole G)
10 bis 100 Gewichtsteilen hydrophile Polyisocyanate H)

Besonders bevorzugt werden die Komponenten A) bis H) in folgenden Mengen eingesetzt:
100 Gewichtsteile isocyanatfunktioneller Präpolymere A)
5 bis 15 Gewichtsteile heterocyclischer Oligomere B)
1 bis 60 Gewichtsteile Wasser C)
0 bis 0,5 Gewichtteile an Katalysatoren D)
0,1 bis 1 Gewichtteile an C₈- bis C₁₂-Monocarbonsäuren oder deren Ammonium- oder Alkalisalze oder C₁₂- bis C₄₄-Dicarbonsäuren oder deren Ammonium- oder Alkalisalze E)
0 Gewichtsteile Tenside F)
0 Gewichtsteile Alkohole G)
20 bis 80 Gewichtsteilen hydrophile Polyisocyanate H)

Die Herstellung der erfindungsgemäßen hydrophilen, aliphatischen Polyurethan-Schäume erfolgt durch Mischen der Komponenten A), C), H) und gegebenenfalls B), D), E), F), G) in beliebiger Reihenfolge, Aufschäumen der Mischung und Aushärtung, bevorzugt durch chemische Vernetzung. Bevorzugt werden die Komponenten A), B) und H) miteinander vorvermischt. Die gegebenenfalls einzusetzenden Carboxylate E) und gegebenenfalls die Tenside F) werden bevorzugt in Form ihrer wässrigen Lösungen dem Reaktionsgemisch zugesetzt.

Das Aufschäumen kann dabei grundsätzlich durch das bei der Reaktion der Isocyanatgruppen mit Wasser gebildete Kohlendioxid erfolgen, die Verwendung von weiteren Treibmitteln ist jedoch ebenfalls möglich. So können prinzipiell auch Treibmittel aus der Klasse der Kohlenwasserstoffe wie C₃C₆-Alkane, z.B. Butane, n-Pentan, *iso*-Pentan, *cyclo*-Pentan, Hexane o.ä. oder halogenierte Kohlenwasserstoffe wie Dichlormethan, Dichlormonofluormethan, Chlordifluorethane, 1,1-Dichlor-2,2,2-Trifluorethan, 2,2-Dichlor-2-fluorethan, insbesondere chlorfreie Fluorkohlenwasserstoffe wie Difluormethan, Trifluormethan, Difluorethan, 1,1,1,2-Tetrafluorethan, Tetrafluorethan (R 134 oder R 134a), 1,1,1,3,3-Pentafluorpropan (R 245 fa), 1,1,1,3,3,3-Hexafluopropan (R 256), 1,1,1,3,3-Pentafluorbutan (R 365 mfc), Heptafluopropan oder auch Schwefelhexafluorid verwendet werden. Auch Gemische dieser Treibmittel sind verwendbar.

Die anschließende Aushärtung erfolgt typischerweise bei Raumtemperatur.

Ein weiterer Gegenstand der vorliegenden Erfindung sind die erfindungsgemäßen Zusammensetzungen sowie die daraus erhältlichen hydrophilen, aliphatischen Polyurethan-Schäume.

Gegenstand der vorliegenden Erfindung sind überdies die nach dem erfindungsgemäßen Verfahren hergestellten Polyurethan-Schäume, sowie die Verwendung der hydrophilen, aliphatischen Polyurethan-Schäume als Bestandteil einer Wundauflage, eines kosmetischen Artikels oder eines Inkontinenzprodukts.

Die Polyurethan-Schäume weisen eine poröse, zumindest teilweise offenzellige Struktur mit miteinander kommunizierenden Zellen auf. Die Dichte der Polyurethan-Schäume liegt dabei typischerweise bei 0,01 bis 0,5 g/cm³ (Bestimmung nach DIN 53420).

Das Absorptionsvermögen gegenüber physiologischer Salzlösung beträgt bei den PolyurethanSchäumen typischerweise 25 bis 150 g pro 100 cm² bei einem 5 mm dicken Schaum. Die Messung erfolgt dabei nach folgendem Verfahren: (Bestimmung nach DIN EN 13726-1, Teil 3.2)

Im Vergleich zu anderen hydrophilen Schäumen kann mit den erfindungsgemäßen PolyurethanSchäumen auch ohne die Verwendung von superabsorbierenden Polymeren eine sehr hohe Absorption von physiologischer Salzlösung erreicht werden. Selbstverständlich ist die Einarbeitung von Superabsorbern aber auch bei den erfindungsgemäßen Polyurethan-Schäumen möglich.

Die Polyurethan-Schäume weisen eine gute mechanische Festigkeit und hohe Elastizität auf. Typischerweise sind die Werte für die Zugfestigkeit größer als 40 kPa, für die Bruchdehnung größer als 30 % und für die Rückprallelastizität größer als 60 % (Bestimmung nach DIN 53504, DIN 53455, DIN EN ISO 3386-1).

Nach der Herstellung können die Polyurethan-Schäume nach an sich bekannten Verfahren zu flächigen Materialien verarbeitet werden, welche dann beispielsweise als Bestandteil einer Wundauflage, eines kosmetischen Artikels oder eines Inkontinenzprodukts eingesetzt werden können. In der Regel werden dazu Blockschäume nach gängigen Methoden auf die gewünschte Dicke geschnitten wodurch flächige Materialien mit einer Dicke von typischerweise von 10 µm bis 5 cm, bevorzugt von 0,1 mm bis 1 cm, besonders bevorzugt von 0,1 mm bis 6 mm, ganz besonders bevorzugt von 0,2 mm bis 6 mm zu erhalten werden.

Mit Hilfe geeigneter Gießtechniken können die beschriebenen flächigen Materialien aber auch direkt durch Auftrag und Aufschäumen der erfindungsgemäßen Zusammensetzung auf ein Substrat, z.B. ein gegebenenfalls vorbehandeltes Papier oder Textil, erhalten werden.

In einer bevorzugten Variante wird dazu eine Mischung der Ausgangsmaterialien, wie in der noch unveröffentlichten europäischen Anmeldung mit der Nummer 09009202.4 beschrieben, mittels eines Rakels auf ein Substrat aufgebracht, woraufhin im Anschluss an das Rakeln das Aufschäumen erfolgt. Die Spalthöhe des Rakels liegt im Allgemeinen im Bereich von 0,2 bis 20 mm, bevorzugt von 0,5 bis 5 und ganz besonders bevorzugt von 0,8 bis 2 mm. Die Filmbreite des zu verwendenden Rakels kann dem jeweiligen Verwendungszweck angepasst werden. Beispiele sind Filmbreiten zwischen 10 und 5000 mm, bevorzugt zwischen 20 und 2000 mm.

Die Polyurethan-Schäume enthalten in der Regel einen nur geringen mit Wasser extrahierbaren Anteil von maximal 2 Gew.-%, bevorzugt von maximal 1 Gew.-%, d.h. sie enthalten nur sehr geringe Mengen an chemisch nicht gebundenen Bestandteilen.

Die Polyurethan-Schäume können überdies mit weiteren Materialien beispielsweise auf Basis von Hydrogelen, (semi-) permeablen Folien, Schaumfolien, Beschichtungen, Hydrokolloiden oder anderen Schäumen verklebt, laminiert oder beschichtet werden.

Die erfindungsgemäßen Polyurethan-Schäume sind besonders zur Herstellung von Wundauflagen geeignet. Dabei können die Polyurethan-Schäume in direktem oder indirektem Kontakt mit der Wunde sein. Bevorzugt werden die Polyurethan-Schäume jedoch in direktem Kontakt mit der Wunde eingesetzt, um beispielsweise eine optimale Absorption von Wundflüssigkeit zu gewährleisten. Die Polyurethan-Schäume zeigen keine Zelltoxizität (Bestimmung nach ISO 10993-5 und ISO 10993-12).

Die Polyurethan-Schäume, die als Wundauflage eingesetzt werden, müssen zusätzlich noch in einem weiteren Verfahrensschritt sterilisiert werden. Zur Sterilisation kommen die dem Fachmann an sich bekannten Verfahren zum Einsatz, bei denen eine Sterilisation durch thermische Behandlung, chemische Stoffe wie Ethylenoxid oder Bestrahlung, beispielsweise durch Gammabestrahlung, erfolgt. Die Bestrahlung kann dabei gegebenenfalls unter Schutzgasatmosphäre erfolgen. Die erfindungsgemäßen Polyurethan-Schäume haben dabei den großen Vorteil, dass sie sich bei Bestrahlung, insbesondere bei Bestrahlung mit Gammastrahlen, nicht verfärben.

Ebenfalls möglich ist die Zugabe, Einarbeitung oder Beschichtung von bzw. mit antimikrobiellen oder biologischen Wirkstoffen, welche sich beispielsweise in Bezug auf die Wundheilung und die Vermeidung von Keimbelastungen positiv auswirken.

### Beispiele

Sofern nicht abweichend gekennzeichnet, beziehen sich alle Prozentangaben auf das Gewicht. Die Bestimmung der Festköpergehalte erfolgte nach DIN-EN ISO 3251. Die Bestimmung der Viskositäten erfolgte bei 23 °C und wurde nach DIN 53019 durchgeführt. Die NCO-Gehalte wurden volumetrisch gemäß DIN-EN ISO 11909 bestimmt.

Als Rakel wurde der Zehntner Universalapplikator ZUA 2000 mit einer Filmbreite von 200 mm und einer Spalthöhe einstellbar von 0 bis 3 mm eingesetzt (Fa. Zehntner GmbH, Sissach, Schweiz).

### Verwendete Substanzen und Abkürzungen

- Desmodur^{®} N 3400:: Aliphatisches Polyisocyanat (HDI-Uretdion), NCO-Gehalt 21,8 %, Bayer MaterialScience AG, Leverkusen, Deutschland
- Desmodur^{®} N 3300:: Aliphatisches Polyisocyanat (BDI-Isocyanurat), NCO-Gehalt 21,8 %, Bayer MaterialScience AG, Leverkusen, Deutschland

### Beispiel 1: Herstellung des Polyurethan-Präpolymers 1 (Baustein A)

Zu einem Gemisch aus 1000 g HDI und 1g Benzoylchlorid wurde bei 80 °C innerhalb von 3 h 1000 g eines Polyalkylenoxids mit einer Molmasse von 4680 g/mol, gestartet auf Glycerin, einem Ethylenoxidgewichtsanteil von 72 % und einen Propylenoxidgewichtsanteil von 28 %, das zuvor bei 100 °C während 6 h bei einem Druck von 0,1 mbar getrocknet wurde, zugetropft und für 12 h nachgerührt. Das überschüssige HDI wurde durch Dünnschichtdestillation bei 130 °C und 0,1 mbar entfernt, wobei die nicht flüchtigen Bestandteile mit 1 g Chlorpropionsäure stabilisiert wurden. Man erhielt ein Präpolymer mit einem NCO-Gehalt von 2,77 % und einer Viskosität von 3500 mPas.

### Beispiel 2: Herstellung des Polyurethan-Präpolymers 2 (Baustein A)

Zu einem Gemisch aus 200 g HDI, 1 g Benzoylchlorid und 1 g Methyltosylat wurde bei 80 °C innerhalb von 2 h 400 g eines Polyalkylenoxids mit einer Molmasse von 5800 g/mol, gestartet auf Glycerin; einem Ethylenoxidgehalt von 80 % und einem Propylenoxidgehalt von 20 %, das vorher bei 100 °C während 6 h bei einem Druck von 0,1 mbar getrocknet wurde, zugetropft und für 12 h nachgerührt. Das überschüssige HDI wurde durch Dünnschichtdestillation bei 130 °C und 0,1 mbar entfernt. Man erhielt ein Präpolymer mit einem NCO-Gehalt von 2,31 % und einer Viskosität von 6070 mPas.

### Beispiel 3 : Herstellung des Polyurethan-Präpolymers 3 (Baustein A)

Zu einem Gemisch aus 1440 g HDI und 4 g Benzoylchlorid wurde bei 80 °C innerhalb von 2 h 2880 g eines Polyalkylenoxids mit einer Molmasse von 4680 g/mol, gestartet auf Glycerin, einem Ethylenoxidgewichtsanteil von 72 % und einem Propylenoxidgewichtsanteil von 28 %, das zuvor bei 100 °C während 6 h bei einem Druck von 0,1 mbar getrocknet wurde, zugetropft und für 1 h nachgerührt. Das überschüssige HDI wurde durch Dünnschichtdestillation bei 130 °C und 0,1 mbar entfernt. Man erhielt ein Präpolymer mit einem NCO-Gehalt von 2,11 % und einer Viskosität von 3780 mPas.

### Beispiel 4: Herstellung des Polyurethan-Präpolymers 4 (Baustein A)

Zu einem Gemisch aus 200 g IPDI, 1g Benzoylchlorid und 1 g Methyltosylat wurde bei 80 °C innerhalb von 2 h 400 g eines Polyalkylenoxids mit einer Molmasse von 5800 g/mol, gestartet auf Glycerin, einem Ethylenoxidgehalt von 80 % und einem Propylenoxidgehalt von 20 %, das vorher bei 100 °C während 6 h bei einem Druck von 0,1 mbar getrocknet wurde, zugetropft und für 12 h nachgerührt. Das überschüssige IPDI wurde durch Dünnschichtdestillation bei 130 °C und 0,1 mbar entfernt. Man erhielt ein Präpolymer mit einem NCO-Gehalt von 2,36 % und einer Viskosität von 8800 mPas.

### Beispiel 5: Herstellung eines hydrophilen Polyisocyanates (Baustein H)

Ein Gemisch aus 282,5 g Desmodur N 3300 und 843,8 g eines Mono-Hydroxy-funktionellen Polyethers auf Ethylenoxid-/Propylenoxidbasis (mit einem Ethylenoxid-Anteil von 80 mol bezogen auf die Gesamtmenge der enthaltenen Oxyalkylengruppen), zahlenmittleres Molekulargewicht 2250 g/mol (OH-Zahl 25 mg KOH/g) wurde in einer Glasapparatur so lang bei 80 °C gerührt, bis der tritrimetrisch ermittelte Gehalt an NCO-Gruppen konstant war. Man erhielt eine Flüssigkeit mit einem NCO-Gehalt von 4,04 % und einer Viskosität von 3330 mPas.

### Beispiel 6: Herstellung eines hydrophilen Polyisocyanates (Baustein H

Ein Gemisch aus 780,0 g Desmodur N 3300 und 500,0 g eines Mono-Hydroxy-funktionellen Polyethers auf Ethylenoxidbasis, zahlenmittleres Molekulargewicht 500 g/mol (OH-Zahl 112 mg KOH/g) wurde in einer Glasapparatur so lang bei 80 °C gerührt, bis der tritrimetrisch ermittelte Gehalt an NCO-Gruppen konstant war. Man erhielt eine Flüssigkeit mit einem NCO-Gehalt von 9,79 % und einer Viskosität von 2510 mPas.

### Beispiel 7: Herstellung eines hydrophilen Polyisocyanates (Baustein H)

Ein Gemisch aus 214,5 g Desmodur N 3300 und 990,0 g eines Mono-Hydroxy-funktionellen Polyethers auf Ethylenoxid-/Propylenoxidbasis (mit einem Ethylenoxid-Anteil von 80 mol bezogen auf die Gesamtmenge der enthaltenen Oxyalkylengruppen), zahlenmittleres Molekulargewicht 2250 g/mol (OH-Zahl 25 mg KOH/g) wurde in einer Glasapparatur so lang bei 80°C gerührt, bis der tritrimetrisch ermittelte Gehalt an NCO-Gruppen konstant war. Man erhielt eine Flüssigkeit mit einem NCO-Gehalt von 2,39 %.

### Beispiel 8: Herstellung eines hydrophilen Polyisocyanates (Baustein H)

Ein Gemisch aus 84,71 g Desmodur N 3300 und 781,9 g eines Mono-Hydroxy-funktionellen Polyethers auf Ethylenoxid-/Propylenoxidbasis (mit einem Ethylenoxid-Anteil von 80 mol bezogen auf die Gesamtmenge der enthaltenen Oxyalkylengruppen), zahlenmittleres Molekulargewicht 2250 g/mol (OH-Zahl 25 mg KOH/g) wird in einer Glasapparatur so lang bei 80 °C gerührt, bis der tritrimetrisch ermittelte Gehalt an NCO-Gruppen konstant ist. Man erhielt eine Flüssigkeit mit einem NCO-Gehalt von 0,29 %.

### Herstellung von Schaumstoffen

Die beiden Isocyanat-Komponenten wurden 15 Sekunden mit einer Rührerdrehzahl von 1200 Upm homogenisiert, dann die weiteren Komponenten eingewogen, weitere 10 Sekunden verrührt und mit einem Rakel (Spalthöhe 1,5 mm) auf silikonisiertes Trennpapier aufgebracht. Als Oligomer wurde, falls verwendet, dabei jeweils Desmodur® N 3400 eingesetzt; als Carboxylat eine 5%ige Lösung von Natriumoleat in Wasser. Darüber hinaus zugesetztes Wasser wird extra angegeben.

| **Komponente [g]** | **Schaumbeispiel** | | | | | | | | |
|---|---|---|---|---|---|---|---|---|---|
| | **1*** | **2** | | **4** | **5** | **6** | **7** | **8** | **9** |
| Präpolymer (Baustein A) aus Bsp.1 | 75g | 60g | 45 g | 45 g | 30 g | 45 g | 30 g | 45 g | 30 g |
| Oligomer | 8,33g | 8,3g | 0 | 8,3 g | 8,3 g | 0 g | 0 g | 0 | 0 |
| Wasser (Baustein C) | 3,35 g | 3,35g | 3,5 g | 3,5 g | 3,5 g | 3,35 g | 45 g | 3,35 g | 3,35 g |
| Hydrophiles PIC (Baustein H) | 0 | 15 g Bsp.5 | 45 g Bsp.5 | 30 g Bsp.5 | 45 g Bsp.5 | 30 g Bsp.6 | 45 g Bsp.6 | 30 g Bsp.7 | 45 g Bsp.7 |
| Natrium-Oleat (Baustein E) | 8,30 g | 8,3 g | 8,2 g | 8,2 g | 8,2 g | 8,3 g | 8,3 g | 8,3 g | 8,3 g |
| als 5 Gew.-%ige Lösung in Wasser | | | | | | | | | |
| | | | | | | | | | |
| Rohdichte [g/1000 cm³] | 174 | 151 | 247 | 141 | 125 | 151 | 120 | 147 | 296 |
| | | | | | | | | | |
| Schaumdicke [mm] | 3,8 | 4,9 | 3,4 | 5,8 | 6,7 | 6,0 | 7,8 | 6,2 | 2,2 |
| Wasseraufnahme [g/100 cm² | 31,4 | 83 | 86 | 106 | 142 | 85 | 98 | 104 | 53 |
| Wasseraufnahme [g/100 cm²] pro mm Schaumdicke | 8,3 | 16,9 | 25,3 | 18,3 | 21,2 | 14,2 | 12,6 | 16,8 | 17,9 |

| | | | | | | | | | |
|---|---|---|---|---|---|---|---|---|---|
| Oligomer (falls vorhanden):Desmodur N 3400 *: Vergleichbeispiel: Schaum ohne die erfindungswesentliche Komponente H) | | | | | | | | | |

Die erfindungsgemäßen Beispiele 2-9 waren Schäume mit einer sehr gleichmäßigen, feinen Porenstruktur und einer angenehmen, weichen Haptik.

Wie diese Beispiele und das Vergleichsbeispiel verdeutlichen, stellen die ertindungsgemäßen Komponenten H) einen maßgeblichen Zusatzstoff in der beschriebenen Schaumherstellung dar: Ohne diese Komponente kann eine sehr hohe Wasseraufnahme nicht erreicht werden. Dies ist überraschend, da bereits die Komponente A) einen sehr hohen Anteil an hydrophilen Polyethern enthält. Es war nicht vorhersehbar, dass durch Zusatz von Bausteinen der Komponente H) hier eine Verbesserung auftritt. Auch die vorteilhafte haptische Beurteilung der erfindungsgemäßen Schäume war nicht absehbar.

## Patentansprüche

1. Verfahren zur Herstellung hydrophiler, aliphatischer Polyurethan-Schäume, bei dem Zusammensetzungen umfassend
A) isocyanatfunktionelle Präpolymere mit einem Gewichtsanteil an niedermolekularen, aliphatischen Diisocyanaten mit einer Molmasse von 140 bis 278 g/mol von unter 1,0 Gew.-% bezogen auf das Präpolymer, erhältlich durch Umsetzung von
A1) niedermolekularen, aliphatischen Diisocyanaten einer Molmasse von 140 bis 278 g/mol mit
A2) di- bis hexafunktionellen Polyalkylenoxiden einer OH-Zahl von 22,5 bis 112 mg KOH/g, und einem Ethylenoxidanteil von 50 bis 100 mol% bezogen auf die Gesamtmenge der enthaltenen Oxyalkylengruppen,
B) gegebenenfalls heterocyclische, 4-Ring- oder 6-Ring-Oligomere von niedermolekularen, aliphatischen Diisocyanaten mit einer Molmasse von 140 bis 278 g/mol,
C) Wasser,
D) gegebenenfalls Katalysatoren,
E) gegebenenfalls C₈- bis C₂₂-Monocarbonsäuren oder deren Ammonium- oder Alkalisalze oder C₁₂- bis C₄₄-Dicarbonsäuren oder deren Ammonium- oder Alkalisalze,
F) gegebenenfalls Tenside und
G) gegebenenfalls ein- oder mehrwertige Alkohole
H) hydrophile Polyisocyanate erhältlich durch Umsetzung von
H1) niedermolekularen, aliphatischen Diisocyanaten einer Molmasse von 140 bis 278 g/mol und / oder daraus herstellbaren Polyisocyanaten mit einer Isocyanatfunctionalität von 2 bis 6 mit
H2) monofunktionellen Polyalkylenoxiden einer OH-Zahl von 10 bis 250, und einem Ethylenoxidanteil von 50 bis 100 mol% bezogen auf die Gesamtmenge der enthaltenen Oxyalkylengruppen.
bereitgestellt, aufgeschäumt und ausgehärtet werden.

2. Verfahren nach Anspruch 1, **dadurch gekennzeichnet, dass** die monofunktionellen Polyalkylenoxide H2) eine OH-Zahl von 20 bis 112 aufweisen.

3. Verfahren nach einem der Ansprüche 1 oder 2, **dadurch gekennzeichnet, dass** die monofunktionellen Polyalkylenoxide H2) einem Ethylenoxidanteil von 60 bis 90 mol% bezogen auf die Gesamtmenge der enthaltenen Oxyalkylengruppen aufweisen.

4. Verfahren nach einem der Ansprüche 1 bis 3, **dadurch gekennzeichnet, dass** der NCO-Gehalt der isocyanatfunktionellen Präpolymere A) 1,5 bis 3,0 Gew.-% beträgt.

5. Verfahren nach einem der Ansprüche 1 bis 4, **dadurch gekennzeichnet, dass** in A1) ausschließlich Hexamethylendiisocyanat (HDI), Isophorondiiocyanat (IPDI), oder deren Mischungen untereinander eingesetzt werden.

6. Verfahren gemäß einem der Ansprüche 1 bis 5, **dadurch gekennzeichnet, dass** in A2) als Polyalkylenoxide Copolymere aus Ethylenoxid und Propylenoxid mit einem Ethylenoxidgehalt, bezogen auf die Gesamtmenge der enthaltenen Oxyalkylengruppen, von 60 bis 85 mol%, gestartet auf Polyolen oder Aminen eingesetzt werden.

7. Verfahren gemäß einem der Ansprüche 1 bis 6, **dadurch gekennzeichnet, dass** die Polyalkylenoxide der Komponente A2) zahlenmittlere Molekulargewichte von 3000 bis 8500 g/mol aufweisen.

8. Verfahren gemäß einem der Ansprüche 1 bis 7, **dadurch gekennzeichnet, dass** die Polyalkylenoxide der Komponente A2) OH-Funktionalitäten von 3 bis 4 aufweisen.

9. Verfahren gemäß einem der Ansprüche 1 bis 8, **dadurch gekennzeichnet, dass** in Komponente B) heterocyclische 4-Ring-Oligomere eingesetzt werden.

10. Verfahren gemäß einem der Ansprüche 1 bis 9, **dadurch gekennzeichnet, dass** als Katalysatoren der Komponente D) Metallsalze, Amine, Amidine und Guanidine eingesetzt werden.

11. Verfahren gemäß einem der Ansprüche 1 bis 10, **dadurch gekennzeichnet, dass** die Komponenten A) bis H) in den folgenden Mengen eingesetzt werden:
100 Gewichtsteile isocyanatfunktioneller Präpolymere A)
0 bis 30 Gewichtsteile heterocyclischer Oligomere B)
0,1 bis 200 Gewichtsteile Wasser C)
0 bis 1 Gewichtteile an Katalysatoren D)
0 bis 5 Gewichtteile an C₈- bis C₁₂-Monocarbonsäuren oder deren Ammonium- oder Alkalisalze oder C₁₂- bis C₄₄-Dicarbonsäuren oder deren Ammonium- oder Alkalisalze E) 0 bis 10 Gewichtsteile Tenside F)
0 bis 20 Gewichtsteile Alkohole G)
5 bis 250 Gewichtsteilen hydrophile Polyisocyanate H)

12. Zusammensetzungen umfassend
A) isocyanatfunktionelle Präpolymere mit einem Gewichtsanteil an niedermolekularen, aliphatischen Diisocyanaten mit einer Molmasse von 140 bis 278 g/mol von unter 1,0 Gew.% bezogen auf das Präpolymer, erhältlich durch Umsetzung von
A1) niedermolekularen, aliphatischen Diisocyanaten einer Molmasse von 140 bis 278 g/mol mit
A2) di- bis hexafunktionellen, bevorzugt tri- bis hexafunktionellen Polyalkylenoxiden einer OH-Zahl von 22,5 bis 112 und einem Ethylenoxidanteil von 50 bis 100 mol% bezogen auf die Gesamtmenge der enthaltenen Oxyalkylengruppen,
B) gegebenenfalls heterocyclische, 4-Ring- oder 6-Ring-Oligomere von niedermolekularen, aliphatischen Diisocyanaten mit einer Molmasse von 140 bis 278 g/mol,
C) Wasser,
D) gegebenenfalls Katalysatoren,
E) gegebenenfalls C₈- bis C₂₂-Monocarbonsäuren oder deren Ammonium- oder Alkalisalze oder C₁₂- bis C₄₄-Dicarbonsäuren oder deren Ammonium- oder Alkalisalze,
F) gegebenenfalls Tenside und
G) gegebenenfalls ein- oder mehrwertige Alkohole
H) hydrophile Polyisocyanate erhältlich durch Umsetzung von
H1) niedermolekularen, aliphatischen Diisocyanaten einer Molmasse von 140 bis 278 g/mol und / oder daraus herstellbaren Polyisocyanaten mit einer Isocyanatfunktionalität von 2 bis 6 mit
H2) monofunktionellen Polyalkylenoxiden einer OH-Zahl von 10 bis 250, und einem Ethylenoxidanteil von 50 bis 100 mol% bezogen auf die Gesamtmenge der enthaltenen Oxyalkylengruppen.

13. Polyurethanschäume erhältlich nach einem Verfahren gemäß einem der Ansprüche 1 bis 11 oder aus Zusammensetzungen gemäß Anspruch 12.

14. Wundauflagen, kosmetische Artikel oder Inkontinenzprodukte erhältlich unter Verwendung von Polyurethanschäumen gemäß Anspruch 13.

## Claims

1. Process for preparing hydrophilic aliphatic polyurethane foams wherein compositions comprising
A) isocyanate-functional prepolymers having a weight fraction of low molecular weight aliphatic diisocyanates having a molar mass of 140 to 278 g/mol of below 1.0% by weight based on the prepolymer, obtainable by reaction of
A1) low molecular weight aliphatic diisocyanates having a molar mass of 140 to 278 g/mol with
A2) di- to hexafunctional polyalkylene oxides having an OH number of 22.5 to 112 mg KOH/g and an ethylene oxide content of 50 to 100 mol% based on the total amount of oxyalkylene groups present,
B) optionally heterocyclic 4-ring or 6-ring oligomers of low molecular weight aliphatic diisocyanates having a molar mass of 140 to 278 g/mol,
C) water,
D) optionally catalysts,
E) optionally C₈-C₂₂ monocarboxylic acids or their ammonium or alkali metal salts or C₁₂-C44 dicarboxylic acids or their ammonium or alkali metal salts,
F) optionally surfactants, and
G) optionally mono- or polyhydric alcohols,
H) hydrophilic polyisocyanates obtainable by reaction of
H1) low molecular weight aliphatic diisocyanates having a molar mass of 140 to 278 g/mol and/or polyisocyanates obtainable therefrom with an isocyanate functionality of 2 to 6, with
H2) monofunctional polyalkylene oxides having an OH number of 10 to 250, and an ethylene oxide content of 50 to 100 mol% based on the total amount of oxyalkylene groups present,
are provided, foamed and cured.

2. Process according to Claim 1, **characterized in that** the monofunctional polyalkylene oxides H2) have an OH number of 20 to 112.

3. Process according to either of Claims 1 and 2, **characterized in that** the monofunctional polyalkylene oxides H2) have an ethylene oxide content of 60 to 90 mol% based on the total amount of oxyalkylene groups present.

4. Process according to any one of Claims 1 to 3, **characterized in that** the NCO content of the isocyanate-functional prepolymers A) is 1.5% to 3.0% by weight.

5. Process according to any one of Claims 1 to 4, **characterized in that** A1) exclusively utilizes hexamethylene diisocyanate (HDI), isophorone diisocyanate (IPDI) or mixtures therebetween.

6. Process according to any one of Claims 1 to 5, **characterized in that** polyalkylene oxides A2) comprise copolymers of ethylene oxide and propylene oxide having an ethylene oxide content, based on the total amount of oxyalkylene groups present, of 60 to 85 mol% and started on polyols or amines.

7. Process according to any one of Claims 1 to 6, **characterized in that** the polyalkylene oxides of component A2) have number average molecular weights of 3000 to 8500 g/mol.

8. Process according to any one of Claims 1 to 7, **characterized in that** the polyalkylene oxides of A2) have OH functionalities of 3 to 4.

9. Process according to any one of Claims 1 to 8, **characterized in that** heterocyclic 4-ring oligomers are used in component B).

10. Process according to any one of Claims 1 to 9, **characterized in that** metal salts, amines, amidines and guanidines are used as catalysts of component D).

11. Process according to any one of Claims 1 to 10, **characterized in that** components A) to H) are used in the following amounts:
100 parts by weight of isocyanate-functional prepolymers A)
0 to 30 parts by weight of heterocyclic oligomers B)
0.1 to 200 parts by weight of water C)
0 to 1 part by weight of catalysts D)
0 to 5 parts by weight of C₈-C₁₂ monocarboxylic acids
or their ammonium or alkali metal salts or C₁₂-C₄₄ dicarboxylic acids or their ammonium or alkali metal salts E),
0 to 10 parts by weight of surfactants F)
0 to 20 parts by weight of alcohols G)
5 to 250 parts by weight of hydrophilic polyisocyanates H)

12. Compositions comprising
A) isocyanate-functional prepolymers having a weight fraction of low molecular weight aliphatic diisocyanates having a molar mass of 140 to 278 g/mol of below 1.0% by weight based on the prepolymer, obtainable by reaction of
A1) low molecular weight aliphatic diisocyanates having a molar mass of 140 to 278 g/mol with
A2) di- to hexafunctional polyalkylene oxides having an OH number of 22.5 to 112 and an ethylene oxide content of 50 to 100 mol% based on the total amount of oxyalkylene groups present,
B) optionally heterocyclic 4-ring or 6-ring oligomers of low molecular weight aliphatic diisocyanates having a molar mass of 140 to 278 g/mol,
C) water,
D) optionally catalysts,
E) optionally C₈-C₂₂ monocarboxylic acids or their ammonium or alkali metal salts or C₁₂-C₄₄ dicarboxylic acids or their ammonium or alkali metal salts,
F) optionally surfactants, and
G) optionally mono- or polyhydric alcohols,
H) hydrophilic polyisocyanates obtainable by reaction of
H1) low molecular weight aliphatic diisocyanates having a molar mass of 140 to 278 g/mol and/or polyisocyanates obtainable therefrom with an isocyanate functionality of 2 to 6, with
H2) monofunctional polyalkylene oxides having an OH number of 10 to 250, and an ethylene oxide content of 50 to 100 mol% based on the total amount of oxyalkylene groups present.

13. Polyurethane foams obtainable by following a process according to any one of Claims 1 to 11 or from compositions according to Claim 12.

14. Wound dressings, cosmetic articles or incontinence products obtainable using polyurethane foams according to Claim 13.

## Revendications

1. Procédé pour la préparation de mousse de polyuréthane aliphatique, hydrophile, dans lequel on prépare, fait mousser et durcit des compositions comprenant
A) des prépolymères à fonctionnalité isocyanate présentant une proportion pondérale de diisocyanates aliphatiques de bas poids moléculaire, présentant une masse molaire de 140 à 278 g/mole, inférieure à 1,0% en poids par rapport au prépolymère, pouvant être obtenus par transformation
A1) de diisocyanates aliphatiques de bas poids moléculaire, présentant une masse molaire de 140 à 278 g/mole avec
A2) des poly(oxydes d'alkylène) difonctionnels à hexafonctionnels présentant un indice d'OH de 22,5 à 112 mg de KOH/g et une proportion d'oxyde d'éthylène de 50 à 100% en mole par rapport à la quantité totale des groupes d'oxyalkylène contenus,
B) des oligomères de 4 ou 6 cycles, le cas échéant hétérocycliques, de diisocyanates aliphatiques de bas poids moléculaire présentant une masse molaire de 140 à 278 g/mole,
C) de l'eau,
D) le cas échéant des catalyseurs,
E) le cas échéant des acides C₈-C₂₂-monocarboxyliques ou leurs sels d'ammonium ou de métal alcalin ou des acides C₁₂-C₄₄-dicarboxyliques ou leurs sels d'ammonium ou de métal alcalin,
F) le cas échéant des agents tensioactifs et
G) le cas échéant des alcools monovalents ou polyvalents
H) des polyisocyanates hydrophiles pouvant être obtenus par transformation
H1) de diisocyanates aliphatiques de bas poids moléculaire, présentant une masse molaire de 140 à 278 g/mole et/ou de polyisocyanates pouvant être préparés à partir de ceux-ci présentant une fonctionnalité isocyanate de 2 à 6 avec
H2) des poly(oxydes d'alkylène) monofonctionnels présentant un indice d'OH de 10 à 250 et une proportion d'oxyde d'éthylène de 50 à 100% en mole par rapport à la quantité totale des groupes d'oxyalkylène contenus.

2. Procédé selon la revendication 1, **caractérisé en ce que** les poly(oxydes d'alkylène) monofonctionnels H2) présentent un indice d'OH de 20 à 112.

3. Procédé selon l'une quelconque des revendications 1 ou 2, **caractérisé en ce que** les poly(oxydes d'alkylène) H2) monofonctionnels présentent une proportion d'oxyde d'éthylène de 60 à 90% en mole par rapport à la quantité totale de groupes d'oxyalkylène contenus.

4. Procédé selon l'une quelconque des revendications 1 à 3, **caractérisé en ce que** la teneur en NCO des prépolymères à fonctionnalité isocyanate A) vaut 1,5 à 3,0% en poids.

5. Procédé selon l'une quelconque des revendications 1 à 4, **caractérisé en ce qu'**on utilise, dans A1), exclusivement du diisocyanate d'hexaméthylène (HDI), du diisocyanate d'isophorone (IPDI) ou leurs mélanges.

6. Procédé selon l'une quelconque des revendications 1 à 5, **caractérisé en ce qu'**on utilise dans A2), comme poly(oxydes d'alkylène), des copolymères d'oxyde d'éthylène et d'oxyde de propylène présentant une teneur en oxyde d'éthylène, par rapport à la quantité totale des groupes d'oxyalkylène contenus, de 60 à 85% en mole, initiés sur des polyols ou des amines.

7. Procédé selon l'une quelconque des revendications 1 à 6, **caractérisé en ce que** les poly(oxydes d'alkylène) du composant A2) présentent des poids moléculaires numériques moyens de 3000 à 8500 g/mole.

8. Procédé selon l'une quelconque des revendications 1 à 7, **caractérisé en ce que** les poly(oxydes d'alkylène) du composant A2) présentent des fonctionnalités OH de 3 à 4.

9. Procédé selon l'une quelconque des revendications 1 à 8, **caractérisé en ce qu'**on utilise, dans le composant B), des oligomères à 4 cycles, hétérocycliques.

10. Procédé selon l'une quelconque des revendications 1 à 9, **caractérisé en ce qu'**on utilise, comme catalyseurs du composant D), des sels métalliques, des amines, des amidines et des guanidines.

11. Procédé selon l'une quelconque des revendications 1 à 10, **caractérisé en ce que** les composants A) à H) sont utilisés dans les quantités suivantes :
100 parties en poids de prépolymères à fonctionnalité isocyanate A),
0 à 30 parties en poids d'oligomères hétérocycliques B),
0,1 à 200 parties en poids d'eau C),
0 à 1 partie en poids de catalyseurs D),
0 à 5 parties en poids d'acides C₈-C₁₂-monocarboxyliques ou leurs sels d'ammonium ou de métal alcalin ou d'acides C₁₂-C₄₄-dicarboxyliques ou leurs sels d'ammonium ou de métal alcalin E),
0 à 10 parties en poids d'agents tensioactifs F),
0 à 20 parties en poids d'alcools G),
5 à 250 parties en poids de polyisocyanates hydrophiles H).

12. Compositions comprenant
A) des prépolymères à fonctionnalité isocyanate présentant une proportion pondérale de diisocyanates aliphatiques de bas poids moléculaire, présentant une masse molaire de 140 à 278 g/mole, inférieure à 1,0% en poids par rapport au prépolymère, pouvant être obtenus par transformation
A1) de diisocyanates aliphatiques de bas poids moléculaire, présentant une masse molaire de 140 à 278 g/mole avec
A2) des poly(oxydes d'alkylène) difonctionnels à hexafonctionnels, de préférence trifonctionnels à hexafonctionnels, présentant un indice d'OH de 22,5 à 112 et une proportion d'oxyde d'éthylène de 50 à 100% en mole par rapport à la quantité totale des groupes d'oxyalkylène contenus,
B) des oligomères de 4 ou 6 cycles, le cas échéant hétérocycliques, de diisocyanates aliphatiques de bas poids moléculaire présentant une masse molaire de 140 à 278 g/mole,
C) de l'eau,
D) le cas échéant des catalyseurs,
E) le cas échéant des acides C₈-C₂₂-monocarboxyliques ou leurs sels d'ammonium ou de métal alcalin ou des acides C₁₂-C₄₄-dicarboxyliques ou leurs sels d'ammonium ou de métal alcalin,
F) le cas échéant des agents tensioactifs et
G) le cas échéant des alcools monovalents ou polyvalents
H) des polyisocyanates hydrophiles pouvant être obtenus par transformation
H1) de diisocyanates aliphatiques de bas poids moléculaire, présentant une masse molaire de 140 à 278 g/mole et/ou de polyisocyanates pouvant être préparés à partir de ceux-ci présentant une fonctionnalité isocyanate de 2 à 6 avec
H2) des poly(oxydes d'alkylène) monofonctionnels présentant un indice d'OH de 10 à 250 et une proportion d'oxyde d'éthylène de 50 à 100% en mole par rapport à la quantité totale des groupes d'oxyalkylène contenus.

13. Mousse de polyuréthane pouvant être obtenue selon un procédé selon l'une quelconque des revendications 1 à 11 ou à partir de compositions selon la revendication 12.

14. Pansements, articles cosmétiques ou produits d'incontinence pouvant être obtenus en utilisant des mousses de polyuréthane selon la revendication 13.
